Europäisches Patentamt

European Patent Office

Office européen des brevets

(11) Publication number: **0 316 355 B1**

## EUROPEAN PATENT SPECIFICATION

(45) Date of publication of patent specification: **26.10.94**

(51) Int. Cl.5: **C07C 1/00**, C07C 4/00, C07C 45/00, C07H 1/00, C07H 1/06, A23L 1/221, A23L 2/26

(21) Application number: **87905371.8**

(22) Date of filing: **29.07.87**

(86) International application number: **PCT/US87/01852**

(87) International publication number: **WO 88/00935 (11.02.88 88/04)**

The file contains technical information submitted after the application was filed and not included in this specification

(54) **PYROLYSIS OF BIOMASS TO PRODUCE MAXIMUM LIOUID YIELDS.**

(30) Priority: **30.07.86 CA 514974**

(43) Date of publication of application:
**24.05.89 Bulletin 89/21**

(45) Publication of the grant of the patent:
**26.10.94 Bulletin 94/43**

(84) Designated Contracting States:
**AT BE CH DE FR GB IT LI NL SE**

(56) References cited:
**GB-A- 1 398 858**
**US-A- 3 298 928**

**Industrial and Engineering Chemistry Process Design and Development, 1985, 24, 581-588.**

**Canadian Journal of Chemical Engineering, 1984, 62, 404-412**

(73) Proprietor: **SCOTT, Donald S.**
**382 Arden Place**
**Waterloo, Ontario N2L 2N7 (CA)**

(72) Inventor: **SCOTT, Donald S.**
**382 Arden Place**
**Waterloo, Ontario N2L 2N7 (CA)**

(74) Representative: **Warren, Anthony Robert et al**
**BARON & WARREN,**
**18 South End,**
**Kensington**
**London W8 5BU (GB)**

**Description**

This invention relates to a process for producing maximum liquid yields from the pyrolysis of biomass and in particular to the manner in which the reaction conditions can be controlled to produce such products as fuel oil, hydroxyacetaldehyde and many other products through the rapid pyrolysis of biomass.

Processes for the rapid pyrolysis of biomass are known. Suitable reactors have been described by Donald S. Scott, et al. in the CANADIAN JOURNAL OF CHEMICAL ENGINEERING, 1984, Volume 62, pp. 404 - 412 and INDUSTRIAL AND ENGINEERING CHEMISTRY PROCESS DESIGN AND DEVELOPMENT, 1985, Volume 24, pp. 581 - 588. Briefly, the reactor described in these publications is a fluidized bed of inert particulate matter as a heating and reacting medium. Other reaction devices that might be suitable are described in U.S. Patent No. 3,853,498 issued December 10th, 1974 naming R.C. Bailie as inventor and U.S. Patent No. 4,510,021 issued April 9th, 1985 and naming Norman K. Sowards as inventor. With previous pyrolysis processes for biomass, it was not known that valuable chemical products could be obtained readily from the liquid product or that the liquid product could be produced in yields sufficiently high to contain concentrations of desirable chemical products in commercial quantity.

It is an object of the present invention to produce in commercial quantities products such as fuel oil, hydroxyacetaldehyde and numerous other products from the rapid pyrolysis of biomass under controlled conditions that can be easily varied depending on the desired product.

According to the invention, there is provided a process of the general type disclosed in the firstmentioned paper by Donald S. Scott et al for producing maximum liquid yields from the pyrolysis of biomass as defined in claim 1.

Reference will now be made to the accompanying drawings, in which:-

Figure 1 is a graph showing the phase relationships for poplar pyrolysis oil and water; and

Figure 2 is a NMR spectra of pyrolitic lignin produced from the process embodying the present invention.

For the purposes of the process embodying the present invention, it is important that the pyrolysis of biomass be conducted rapidly in an appropriate device. Otherwise, the high yields of pyrolysis liquids of the desired composition necessary for economical processing will not be obtained. When the term biomass is used in this specification, it shall be deemed to refer to ligno-cellulosic biomass. All references to percentage in this application are by weight unless otherwise specified.

The gas and vapor residence times are less than ten seconds and, preferably, equal to or less than approximately two seconds. The solid particle residence time is equal to or greater than the gas residence time. Particle size is less than 5 mm and, preferably, equal to or less than approximately 2 mm in its smallest dimension. The temperature of pyrolysis is in the range from substantially 400°C to substantially 650°C and, preferably, in the range from substantially 450°C to substantially 550°C. Atmospheric pressure is satisfactory but pressure significantly above atmospheric can be used also.

The process of the present invention can be used to produce a pyrolysis liquid containing 10% to 30% water with the balance of said liquid being organic compounds including a majority of the lignin type material present in the original biomass together with carbohydrates derived from cellulose. Preferably, the moisture content of the biomass feed ranges from 5% to 15% and the total liquid yield ranges from 50% to 90% and, still more preferably, from 60% to 90%. If rapid pyrolysis of biomass is carried out in accordance with the present invention, a maximum yield of liquid products can be obtained and the liquid can be separated into two fractions. These two fractions can then be further processed to yield valuable products. For example, a pyrolysis liquid can be produced in accordance with the present invention having properties that make it suitable for use as a fuel oil.

The two fractions are created by diluting the pyrolysis liquid obtained by the process with water to a water content of at least 50% and, preferably, at least 55%, at temperatures near ambient. This causes the two fractions to separate and the water-poor fraction can then be filtered off from the water-rich fraction. As an example of a pyrolysis liquid that can be used as a substitute for fuel oil, such a liquid can be obtained from the rapid pyrolysis of a hardwood, for example, poplar wood. The poplar wood preferably contains 5% to 10% moisture although moisture contents as fed up to 30% can be used. The pyrolysis liquid obtained will typically have a composition of approximately 75% to 85% organic liquid and 15% to 25% water depending on the operating conditions. The yield of pyrolysis liquid will be 65% to 75% of the dry wood fed. The liquid will typically have a density ranging from 1,150 kg/m3 to 1,200 kg/m3 and a pH ranging from 2.3 to 3.5. The pyrolysis liquid is a single phase homogeneous liquid in which the water is dissolved in the organic phase. The liquid will have certain significant quantities of chemicals or classes of chemicals if the liquid has been prepared in accordance with the process of the present invention.

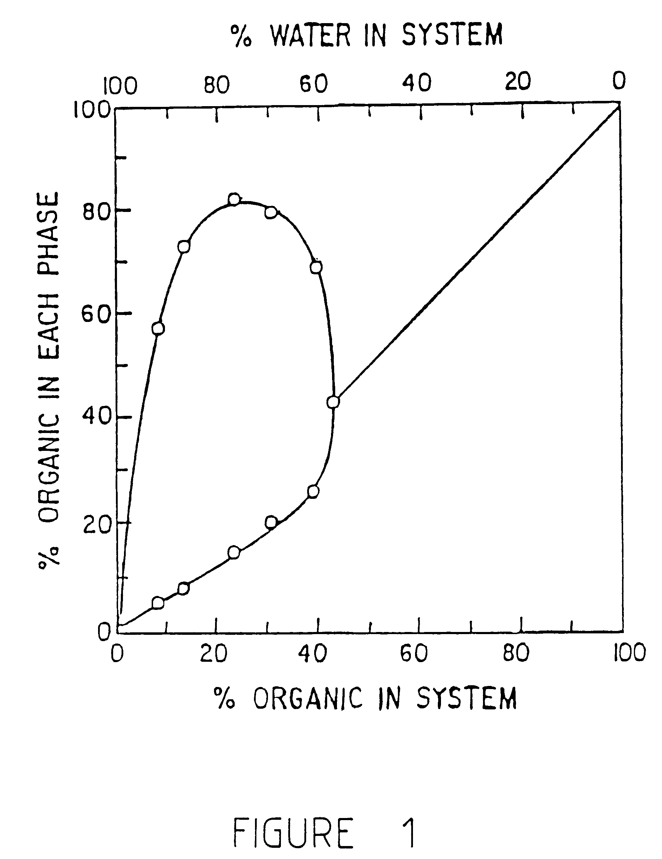

In Figure 1, there is shown a typical solubility diagram for a liquid obtained from poplar wood. Pyrolytic liquids prepared by other methods from wood, which do not meet the process requirements of rapid pyrolysis for maximum liquid production will not show similar solubility behaviour because their chemical composition will be quite different. The phase diagram of Figure 1 shows that if the pyrolysis liquid as produced is diluted with water to a water content of 55% or more at temperatures near ambient, with no further treatment, a water-poor phase will separate and can be filtered off from a water-rich phase. The water-poor phase contains insoluble material that can be dried at temperatures preferably not exceeding 110°C and then easily ground to give a fine free flowing material. The crude pyrolysis liquid can also be heated for a period if desired, before or after dilution with water, in order to cause acid hydrolysis of oligosaccharides or of anhydrosaccharides to occur to increase the yield of monomeric sugar-related carbohydrates. However, this step is not essential to the separation.

The water-poor fraction is primarily derived from the lignin content of the biomass and is largely aromatic in character. Depending on the biomass feed, the water-poor fraction will be 10% to 30% of the dry wood fed, or 15% to 40% of the pyrolysis liquid. Further, it is only under the previously described conditions of rapid pyrolysis that these yields of lignin related materials can be recovered in the pyrolysis liquid and can be readily separated from this liquid.

The water-rich fraction-contains a high proportion of hydroxy carbonyl compounds related to sugars. The water-rich fraction can be further treated so that acids are first removed by any suitable method. Primarily formic and acetic acids will be removed through distillation, neutralization, solvent extraction or ion exchange. However, because yields of these acids are substantial, methods such as distillation or solvent extraction are preferred if the acid is to be recovered as a commercial product. If desirable, treatment with charcoal, or other adsorbent, or extraction with a suitable solvent will largely remove residual amounts of aromatic or furanoid compounds from the aqueous solution. The treated aqueous solution can then be used as a source of carbohydrates for fermentation processes. It can also be used as a feed stock for the recovery of specific chemicals, in particular, the product hydroxyacetaldehyde (glycolaldehyde or 2-hydroxy ethanal), as well as other components present, for example, levoglucosan, acetol, glyoxal, methyl glyoxal and numerous other components. In some processes, the amount of a particular component will not be sufficient to justify its recovery. However, usually the reaction conditions can be varied so that the yield of a particular desirable component will be increased.

In Table 1, there is shown the results for a typical pyrolysis process in an atmospheric pressure fluidised bed reactor of the type described in the papers by Donald S. Scott, et al referred to previously. The letters "m.f." in Table 1 mean moisture free and the yields are provided on a moisture free basis after deducting for the moisture in the feed. In Table 2, there are set out typical yields of the more important compounds present in the water-rich fraction at a particular temperature. The yield of any given component can be maximized by the variation of temperature and residence time in the reactor. Of course, the yield can also be varied with the type of feed stock. It is to be noted particularly in Table 2 the high yield of hydroxyacetaldehyde.

When it is desired to produce a pyrolysis liquid that can be used as a fuel oil, the water content must be adjusted to between 10% and 30% and, preferably, between 15% and 25%. This amount of water, when referring to the phase diagram of Figure 1, dissolves in the organic material. Also, this concentration of water reduces the viscosity of the liquid so that it remains fluid and pumpable (i.e. 40 centipoises at 40°C). The water content also gives the liquid a very low pour point (-24°C for liquid from poplar wood). Further, the water content stabilizes the liquid in storage and storage of liquid in sealed containers shows little change over eighteen months at room temperature. If the water content is too low, the

## TABLE 1

## PILOT PLANT TEST RESULTS

IEA Standard Poplar-Aspen, -595um, 6.25% moisture, 0.44% Ash m.f.
C (mf) 48.39%, H (mf) 5.89%, N - .068%

| Run No. | 30 | 28 | 27 | 25 | 26 | 34* |
|---|---|---|---|---|---|---|
| Vapor Res. Time, secs | 0.616 | 0.584 | 0.550 | 0.539 | 0.520 | 0.539 |
| Temp. in Bed, C | 425 | 465 | 500 | 541 | 625 | 541 |
| Feed Rate, kg/hr | 2.391 | 1.709 | 2.238 | 1.281 | 1.001 | 1.297 |
| Yields, % of Wood mf. | | | | | | |
| Organic Liquids | 55.88 | 67.17 | 65.76 | 63.65 | 40.27 | 62.05 |
| Water (Pyrolytic K.F.) | 3.8 | 5.5 | 9.3 | 7.4 | 4.1 | 7.7 |
| Char | 30.51 | 18.88 | 12.15 | 8.99 | 7.81 | 9.72 |
| Gas | 5.95 | 8.53 | 12.45 | 21.22 | 36.65 | 19.11 |
| $H_2$ | .004 | .009 | .019 | .064 | .235 | .043 |
| CO | 2.06 | 3.27 | 5.32 | 10.47 | 22.88 | 9.13 |
| $CO_2$ | 3.72 | 4.86 | 6.30 | 8.64 | 8.44 | 8.25 |
| $CH_4$ | .091 | .20 | .48 | 1.28 | 3.51 | .89 |
| $C_2H_4$ | .038 | .085 | .20 | .49 | 1.09 | .36 |
| $C_2H_6$ | .010 | .021 | .043 | .12 | .23 | .15 |
| $C_3^+$ | .031 | .085 | .085 | .16 | .27 | .31 |
| Overall Recovery, % | 96.14 | 100.08 | 99.66 | 101.26 | 88.83 | 98.58 |
| Liquid Condensate, gms/ml | 1.145 | 1.171 | 1.172 | 1.130 | 0.992 | 1.158 |

EP 0 316 355 B1

Table 2

| Typical Yields of Some Organic Components of Pyrolysis Oil from Fast Pyrolysis of Poplar Wood at 504°C | |
| --- | --- |
| | % by weight of dry wood |
| Cellobiosan and other anhydrodi saccharides | 1.11 |
| Glucose | 0.55 |
| Fructose | 1.34 |
| Glyoxal | 1.42 |
| Levoglucosan + methylglyoxal | 2.52 |
| Hydroxyacetaldehyde | 6.47 |
| Formic acid | 5.40 |
| Acetic acid | 6.30 |
| Ethylene glycol | 0.87 |
| Acetol (1-hydroxy propanone) | 1.70 |

foregoing advantages disappear. Similarly, if the water content is too high, the heating value of the liquid becomes insignificant and sustained combustion is difficult. In the preferred range of water content, the pyrolysis liquid from wood can be burned with appropriate ignition devices in the same combustion equipment as that used for fuel oils with either pressure atomizing or gas atomizing burners. As a further advantage, the fuel oil obtained from the process of the present invention contains essentially no ash and is very low in sulfur content.

From hardwoods or softwoods, it is possible to obtain a yield of pyrolytic liquids ranging from 70% to 75%. The water-poor fraction is essentially identical to the lignin fraction produced in steam explosion processes. Steam explosion processes are very different and less economical than the process of the present invention. Comparison of the water-poor fraction of the present invention with the lignin derived material from steam explosion processes using nuclear magnetic resonance spectra, shows the two materials are similar.

While the process has been specifically described in the examples using poplar wood, numerous other woods can be used in the process and the conditions can be varied to produce maximum yields of particular components. Variations, within the scope of the attached claims, will be readily apparent to those skilled in the art.

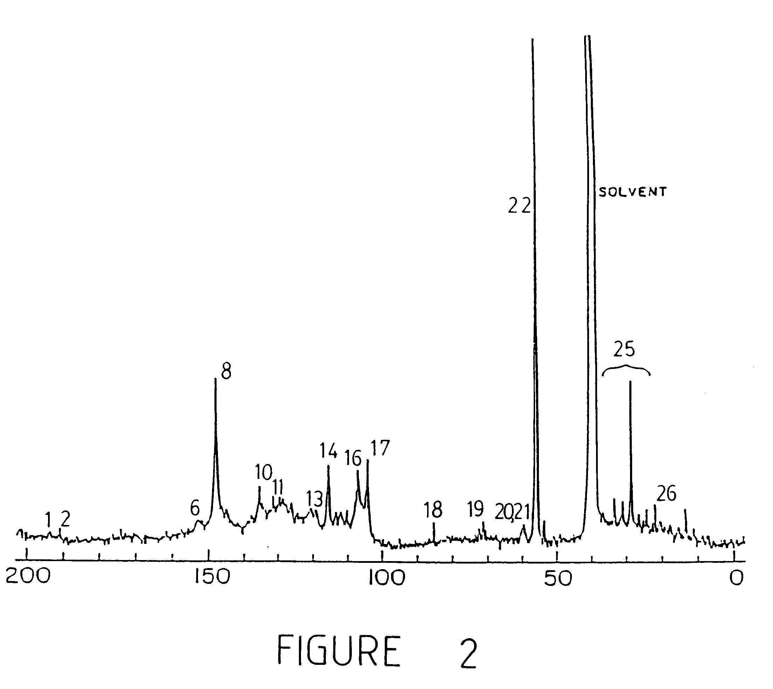

Figure 2 is a NMR spectra of pyrolytic lignin resulting from fast pyrolysis of poplar wood in accordance with the process of the present invention.

**Claims**

1.  A process for producing maximum liquid yields from the pyrolysis of biomass containing lignin-type material and cellulose, by using a reactor capable of rapid heat exchange and short gas residence times, said process introducing said biomass into said reactor, said biomass having a moisture content of 5% to 15% by weight, introducing a gas into said reactor, introducing heat into said reactor and carrying out a pyrolysis process of said biomass to produce solid, liquid and vapor products under the following conditions:

    (a) gas and vapor residence time is less than ten seconds;
    (b) solid particle residence time is equal to or greater than the gas and vapor residence time;
    (c) the solid particle size does not exceed 5 mm;
    (d) the pyrolysis process is carried out at a temperature ranging from substantially 400°C to substantially 650°C; and
    (e) the pressure is near one atmosphere;

    producing a single phase pyrolysis liquid containing 10% to 30% by weight water with the balance of said liquid being organic compounds including a majority of the lignin type material present in the original biomass together with carbohydrates derived from cellulose, the total liquid yield ranging from substantially 56% to substantially 90% by weight, said process being characterized by processing said single phase pyrolysis liquid to divide said liquid into two fractions, namely a water-rich fraction and a water-poor fraction, by diluting the pyrolysis liquid obtained from the process with water to a water content of at least 50% by weight, at temperatures near ambient causing the two fractions to separate, and filtering off the water-poor fraction from the water-rich fraction.

2. A process as claimed in Claim 1 wherein the gas and vapor residence time does not exceed approximately two seconds.

3. A process as claimed in Claim 2 wherein the particle size of the biomass does not exceed substantially 2 mm.

4. A process as claimed in Claim 3 wherein the process is carried out at a temperature ranging from substantially 450°C to substantially 550°C.

5. A process as claimed in Claim 4 wherein the two fractions are created by diluting the pyrolysis liquid obtained from the process with water to a water content of at least 55% by weight.

6. A process as claimed in Claim 1 wherein the water-poor fraction ranges from 10% to 30% by weight of dry wood fed and 15% to 40% by weight of the pyrolysis liquid.

7. A process as claimed in any one of Claims 1 or 5 including recovering the water-poor phases by filtration, washing and drying to yield a lignin-derived material.

8. A process as claimed in Claim 6 wherein the water-rich fraction is further processed to remove acids, and the fraction is then further treated to remove residual amounts of aromatic or furanoid compounds, the product so formed being a source of carbohydrates.

9. A process as claimed in Claim 8 wherein the aromatic or furanoid compounds are removed using adsorption.

10. A process as claimed in Claim 8 wherein the aromatic or furanoid compounds are removed using extraction.

11. A process as claimed in Claim 8 wherein one or more of the following products are recovered from the remaining product of the water-rich phase, namely hydroxyacetaldehyde, levoglucosan, acetol, glyoxal or methyl glyoxal.

12. A process as claimed in any one of Claims 1, 5 or 9 wherein hydroxyacetaldehyde is recovered from the water-rich phase.

13. A process as claimed in Claim 4 wherein the pyrolysis liquid resulting from the pyrolysis process is diluted with water so that the water content ranges between 10% and 30% by weight, said pyrolysis liquid being suitable for use as a fuel oil.

14. A process as claimed in Claim 4 wherein the pyrolysis liquid is diluted with water so that the water content ranges from 15% to 25% by weight, making the pyrolysis liquid suitable for use as a fuel oil.

15. A process as claimed in Claim 4 wherein the biomass is a hardwood or softwood and the yield of pyrolysis liquid ranges from 70% to 75% by weight.

**Patentansprüche**

1. Ein Prozess zum Gewinnen der höchsten Ausbeute von Flüssigkeit aus einer Pyrolyse von Holzmaterial und Zellulose enthaltender Biomasse, durch einen Reaktor der einen schnellen Wärmeaustausch und kurze Gasaufenthaltszeiten ermöglicht, wobei in diesem Prozess die Biomasse in den Reaktor eingebracht wird, und die Biomasse einen Flüssigkeitsgehalt zwischen 5% bis 15% ihres Gewichtsanteils aufweist, und ein Gas in den Reaktor eingebracht wird, und Hitze dem Reaktor zugeführt wird, wobei dann ein Pyrolyse-Prozess der Biomasse ausgeführt wird, um feste, flüssige und dampfförmige Produkte unter den folgenden Bedingungen zu erzeugen:
   (a) die Gas- und Dampfaufenthaltszeit ist kleiner als 10 Sekunden;
   (b) die Festpartikel-Aufenthaltszeit ist gleich oder größer als die Gas- und Dampfaufenthaltszeit;

(c) die Festpartikel-Größe übersteigt nicht 5 mm;

(d) der Pyrolyseprozess findet im wesentlichen bei einer Temperatur zwischen 400°C bis etwa 650°C statt; und

(e) der Druck beträgt etwa 1 Atmosphäre;

wobei eine Einphasen-Pyrolyse-Flüssigkeit entsteht, welche 10% bis 30% Gewichtsanteile Wasser enthält, wobei das Gleichgewicht dieser Flüssigkeit aus organischen Verbindungen einschließlich der Mehrheit des holzförmigen Materials der ursprünglichen Biomasse zusammen mit Kohlenhydraten gewonnen aus Zellulose besteht, wobei die gesamte Flüssigkeitsausbeute im wesentlichen zwischen 56% bis zu 90% Gewichtsanteil beträgt, und dieser Prozess dadurch gekennzeichnet ist, daß die Einphasen-Pyrolyse-Flüssigkeit derart verarbeitet wird, daß die Flüssigkeit in zwei Teile zerlegt wird, nämlich einen wasserreichen Anteil und einen wasserarmen Anteil, dadurch, daß die durch den Prozess erhaltene Pyrolyse-Flüssigkeit mit Wasser bis aus einen Wassergehalt von mindestens 50% Gewichtsanteilen verdünnt wird, und bei einer Temperatur nahe der Umgebungstemperatur die zwei Anteile getrennt werden und der wasserarme Anteil vom wasserreichen Anteil abgefiltert wird.

2. Ein Prozess nach Anspruch 1, wobei die Gas- und Dampf-Aufenthaltsdauer etwa 2 Sekunden nicht überteigt.

3. Ein Prozess nach Anspruch 2, wobei die Partikelgröße der Biomasse im wesentlichen 2 mm nicht übersteigt.

4. Ein Prozess nach Anspruch 3, wobei der Prozess im wesentlichen bei einer Temperatur zwischen 450 bis zu 550°C stattfindet.

5. Ein Prozess nach Anspruch 4, wobei die zwei Anteile durch Verdünnen der durch den Prozess gewonnenen Pyrolyseflüssigkeit mit Wasser auf einen Wassergehalt von mindestens 55% Gewichtsanteil gebildet werden.

6. Ein Prozess nach Anspruch 1, wobei der wasserarme Anteil aus 10% bis 30% Gewichtsanteil trockenen Holzstoffs und 15% bis 40% Gewichtsanteil Pyrolyseflüssigkeit besteht.

7. Ein Prozess nach einem der Ansprüche 1 bis 5, welcher ein Wiedergewinnen der wasserarmen Phase durch Filtration, Waschen und Trocknen einschließt, um ein vom Holzstoff abgeleitetes Material zu erhalten

8. Ein Prozess nach Anspruch 6, wobei der wasserreiche Anteil weiterverarbeitet wird, um Säuren zu entfernen, wobei der Anteil weiter behandelt wird, um zurückbleibende Mengen von aromatischen oder furanoiden Verbindungen zu entfernen, wobei das so gebildete Material der Ursprung der Kohlenhydrate ist.

9. Ein Prozess nach Anspruch 8, wobei die aromatischen oder furanoiden Verbindungen durch Adsorption entfernt werden.

10. Ein Prozess nach Anspruch 8, wobei die aromatischen oder Furanverbindungen durch Extraktion entfernt werden.

11. Ein Prozess nach Anspruch 8, wobei eine oder mehrere der folgenden Produkte aus dem zurückbleibenden Produkt der wasserreichen Phase wiedergewonnen werden, nämlich Hydroxyacetaldehyd, Levoglucosan, Acetol, Glyoxal oder Methyl-Glyoxal.

12. Ein Prozess nach einem der Ansprüche 1, 5 oder 9, wobei Hydroxyacetaldehyd aus der wasserreichen Phase zurückgewonnen wird.

13. Ein Prozess nach Anspruch 4, wobei die Pyrolyseflüssigkeit, entstanden durch den Pyrolyseprozess, mit Wasser verdünnt wird, wo daß der Wassergehalt zwischen 10% und 30% Gewichtsanteil beträgt, wobei diese Pyrolyseflüssigkeit als Heizöl verwendet werden kann.

**14.** Ein Prozess nach Anspruch 4, wobei die Pyrolyseflüssigkeit mit Wasser verdünnt wird, so daß der Wassergehalt zwischen 15% bis 20% Gewichtsanteil beträgt, um die Pyrolyseflüssigkeit als Heizöl gebrauchen zu können.

**15.** Ein Prozess nach Anspruch 4, wobei die Biomasse aus Hartholz oder Weichholz besteht und die Ausbeute von Pyrolyseflüssigkeit zwischen 70% bis 75% Gewichtsanteil beträgt.

**Revendications**

**1.** Procédé pour obtenir des productions maximales de liquides à partir de la pyrolyse de biomasse contenant un matériau du type lignine et de la cellulose, en utilisant un réacteur capable d'échange thermique rapide et de courts temps de séjour de gaz, ledit procédé comprenant d'introduire ladite biomasse dans ledit réacteur, ladite biomasse ayant un taux d'humidité de 5 à 15% en poids, introduire un gaz dans ledit réacteur, introduire de la chaleur dans ledit réacteur et d'effectuer un procédé de pyrolyse de ladite biomasse pour obtenir des produits solides, liquides et vapeurs dans les conditions suivantes :

(a) le temps de séjour du gaz et de la vapeur est inférieur à 10 secondes ;

(b) le temps de séjour des particules solides est égal ou supérieur au temps de séjour du gaz et de la vapeur ;

(c) la taille des particules solides n'excède pas 5 mm ;

(d) le procédé de pyrolyse est effectué à une température comprise entre sensiblement 400°C à sensiblement 650°C ; et

(e) la pression est proche d'une atmosphère ;

produisant un liquide de pyrolyse monophasique contenant 10% à 30% en poids d'eau avec le restant dudit liquide étant des composés organiques comprenant une majorité de matériau du type lignine présent dans la biomasse de départ ensemble avec des hydrates de carbone derivés de la cellulose, la production totale de liquide est comprise entre sensiblement 56% et sensiblement 90% en poids, ledit procédé étant caractérisé en ce que ledit liquide de pyrolyse monophasique est traité pour diviser ledit liquide en deux fractions, notamment une fraction riche et eau et une fraction pauvre en eau, en diluant le liquide de pyrolyse obtenu à partir du procédé avec de l'eau jusqu'à une teneur en eau d'au moins 50% en poids, à des températures proches de la température ambiante, causant la séparation des deux fractions, et en extrayant par filtration la fraction pauvre en eau de la fraction riche en eau.

**2.** Procédé selon la revendication 1, dans lequel le temps de séjour du gaz et de la vapeur n'excède pas deux secondes environ.

**3.** Procédé selon la revendication 2, dans lequel la taille des particules de la biomasse n'excède pas 2 mm environ.

**4.** Procédé selon la revendication 3, dans lequel le procédé est effectué à une température comprise entre sensiblement 450°C et sensiblement 550°C.

**5.** Procédé selon la revendication 4, dans lequel les deux fractions sont créées en diluant le liquide de pyrolyse obtenu à partir du procédé avec de l'eau jusqu'à une teneur en eau d'au moins 55% en poids.

**6.** Procédé selon la revendication 1, dans lequel la fraction pauvre en eau comprend de 10% à 30% en poids du bois sec alimenté et de 15% à 40% en poids du liquide de pyrolyse.

**7.** Procédé selon l'une quelconque des revendications 1 à 5, comprenant de récupérer les phases pauvres en eau par filtration, lavage et séchage pour produire un matériau dérivé de la lignine.

**8.** Procédé selon la revendication 6, dans lequel la fraction riche en eau est en outre traitée pour éliminer les acides, et la fraction est ensuite traitée pour éliminer les quantités résiduelles de composés aromatiques ou furanoïdes, le produit ainsi formé étant une source d'hydrates de carbone.

**9.** Procédé selon la revendication 8, dans lequel les composés aromatiques ou furanoïdes sont éliminés par adsorption.

**10.** Procédé selon la revendication 8, dans lequel les composés aromatiques ou furanoïdes sont éliminés par extraction.

**11.** Procédé selon la revendication 8, dans lequel un ou plusieurs des produits suivants sont récupérés du produit restant de la phase riche en eau, notamment l'hydroxyacetaldehyde, le levoglucosan, l'acétol, le glyoxal ou le glyoxal de méthyle.

**12.** Procédé selon l'une quelconque des revendications 1, 5 ou 9, dans lequel l'hydroxyacetaldehyde est récupéré de la phase riche en eau.

**13.** Procédé selon la revendication 4, dans lequel le liquide de pyrolyse résultant du procédé de pyrolyse est dilué avec de l'eau, de sorte que la teneur en eau est comprise entre 10% et 30% en poids, ledit liquide de pyrolyse étant adapté à l'utilisation en tant que fuel.

**14.** Procédé selon la revendication 4, dans lequel le liquide de pyrolyse est dilué avec de l'eau de sorte que la teneur en eau est comprise entre 15% et 25% en poids, rendant le liquide de pyrolyse apte à l'utilisation en tant que fuel.

**15.** Procédé selon la revendication 4, dans lequel la biomasse est un bois dur ou un bois tendre et la production de liquide de pyrolyse se situe entre 70% et 75% en poids.

FIGURE 1

FIGURE 2